# EUROPEAN PATENT SPECIFICATION

(11) **EP 2 776 082 B1**
(45) Date of publication and mention of the grant of the patent: **08.01.2020**
(21) Application number: 12791370.5
(22) Date of filing: 08.11.2012
(51) Int. Cl.: A61L 27/56, A61L 27/58, A61L 27/38

(54) **METHOD FOR ELIMINATION OF SPACE THROUGH TISSUE APPROXIMATION**
VERFAHREN ZUR RAUMELIMINIERUNG DURCH GEWEBEANNÄHERUNG
MÉTHODE POUR COMBLER LES INTERSTICES DANS LE RAPPROCHEMENT DE TISSUS

(30) Priority: 10.11.2011 US 201161558083 P
(43) Date of publication of application: 17.09.2014
(60) Divisional application: 19204646.4
(73) Proprietor: LifeCell Corporation, Madison, New Jersey 07940 (US)
(72) Inventor: OWENS, Rick, Stewartsville, NJ 08886 (US); XU, Hui, Plainsboro, NJ 08536 (US); SUN, Wenquan, Warrington, PA 18976 (US)
(74) Representative: Ashton, Timothy
(86) International application number: PCT/US2012/064103
(87) International publication number: WO 2013/070881

(56) References cited:
- WO-A1-2005/009134
- WO-A1-2012/142419
- WO-A2-2009/009620
- WO-A2-2010/019753
- WO-A2-2010/078353
- V.K. DEEPAK ET AL.: "Evaluation of closed incision management with negative pressure wound therapy (CIM): Hematoma/seroma and involvement of the lymphatic system", WOUND REPAIR AND REGENERATION, vol. 19, 23 May 2011 (2011-05-23), pages 588-596, XP002690941,

## Description

The present disclosure relates generally to compositions and methods for the repair, regeneration, and/or treatment of damaged or defective tissue. In particular, disclosed are compositions and methods for approximating tissue following surgical procedures that result in tissue separation or otherwise create space between tissue planes. This tissue separation can result in the formation of undesirable seromas and/or hematomas.
WO 2010/019753 A2 discloses devices for treating wounds and methods of treating wounds using tissue scaffolds. V.K. Deepak et al., 2011 discloses the effect of closed incision management with negative pressure wound therapy on hematoma/seroma formation.

Seromas and hematomas are common complications associated with many surgical procedures. A seroma is a pocket of clear or yellow serous fluid originating from serous glands. A hematoma, in contrast, is a localized collection of blood outside a blood vessel. Both can result from trauma, such as a blow or fall, as well as from disease or surgical procedures. Seromas and hematomas are common after plastic surgery, particularly for surgery in the head/neck area, as well as after abdominal surgery.

The formation of seromas and hematomas after surgery can hinder the recovery process. While seromas and hematomas will often resolve without intervention, some patients require repeated follow-up visits to have them drained or otherwise treated. Furthermore, the swelling caused by seromas and hematomas will not always fully subside, leading to the formation of an unsightly knot of calcified tissue that can require additional surgical intervention.

Current treatment for seromas and hematomas consists primarily in the use of drains to remove fluid and the subsequent reduction of dead space. These drainage treatments rely on the passive action of drains, which are often left in place for lengthy periods of time, thereby necessitating extended medical care and increasing the risk of infection. Alternate investigative approaches to eliminating space include the use of glues or other adhesives to bind tissue planes together and thereby reduce the open space available for fluid accumulation. However, these alternatives remain experimental. Thus, a need remains for alternative methods of promoting natural tissue adhesion and of eliminating fluid accumulation within the open space between separated tissue planes.

Accordingly, disclosed herein are acellular tissue matrix compositions, methods of preparing the compositions, and methods of using the compositions to treat or otherwise ameliorate the complications associated with separated tissue resulting from disease, trauma, or surgery.
The invention is defined by the claims.

In various embodiments, a method of approximating separated tissue is provided, the method comprising implanting into a space between two or more separated tissue planes an acellular tissue matrix, wherein the acellular tissue matrix comprises the extracellular matrix of a decellularized tissue. In some embodiments, the implanted acellular tissue matrix comprises particulate acellular tissue fragments, or a foam, film, or other porous material capable of supporting migration and proliferation of cells from surrounding tissue following implantation. In certain embodiments, implanting the acellular tissue matrix prevents formation of a seroma or hematoma within the space between the separated tissue planes. In some embodiments, implanting the acellular tissue matrix promotes faster approximation of the separated tissue planes, as compared to tissue approximation in the absence of an implanted acellular tissue matrix.

In various embodiments, the implanted acellular tissue matrix further comprises at least one biological or non-biological mesh. In some embodiments, the extracellular matrix of the decellularized tissue overlays at least a portion of one surface of the mesh. In other embodiments, the mesh overlays at least a portion of one surface of the extracellular matrix of the decellularized tissue.

In some embodiments, the implanted acellular tissue matrix further comprises one or more viable cells that are histocompatible with the patient in which they are being implanted. In certain embodiments, the histocompatible cells are mammalian cells. In one embodiment, the one or more viable cells are stem cells. In some embodiments, the implanted acellular tissue matrix further comprises at least one additional factor selected from a cell growth factor, an angiogenic factor, a differentiation factor, a cytokine, a hormone, and a chemokine. In certain embodiments, the at least one additional factor is encoded by a nucleic acid sequence contained within an expression vector. In one embodiment, the expression vector is contained within one or more viable cells that are histocompatible with the patient in which they are being implanted.

In various embodiments, the implanted acellular tissue matrix is secured to the surrounding tissue planes. The implanted acellular tissue matrix can be secured to the surrounding tissue planes using biodegradable sutures, positive external pressure to the tissue planes surrounding the implanted acellular tissue matrix, or through a reduced pressure therapy device. In some embodiments, the positive external pressure comprises a dressing or binding around the tissue planes. In other embodiments, the reduced pressure therapy device comprises a negative pressure source that is fluidly connected by a fluid passage or tubing to the acellular tissue matrix and wherein the reduced pressure therapy device delivers negative internal pressure to the acellular tissue matrix.

In some embodiments, the reduced pressure therapy device further comprises a porous manifold that is placed in or near the implanted acellular tissue matrix, is connected to the negative pressure source by the fluid passage or tubing, and distributes negative pressure within or near the implanted acellular tissue matrix. In some embodiments, the negative pressure source is a pump. In certain embodiments, the reduced pressure therapy device further comprises a drape to seal a site where an acellular tissue matrix has been implanted. In one embodiment, the drape is composed of a flexible polymeric material. In another embodiment, the drape is of sufficient thickness to allow for a reduced pressure therapy under the drape. In certain embodiments, an adhesive is applied to the drape to seal the drape to the site where an acellular tissue matrix has been implanted.
In various embodiments, an acellular tissue matrix as described herein for use in a method of treating a patient in need of tissue approximation is provided, comprising implanting into a space between two or more separated tissue planes an acellular tissue matrix, wherein the acellular tissue matrix comprises the extracellular matrix of a decellularized tissue. In some embodiments, the implanted acellular tissue matrix comprises particulate acellular tissue fragments, or a foam, film, or other porous material capable of supporting the migration and proliferation of cells from the separated tissue planes following implantation. In some embodiments, implanting the acellular tissue matrix prevents formation of a seroma or hematoma within the space between the separated tissue planes. In certain embodiments, implanting the acellular tissue matrix promotes faster approximation of the separated tissue planes, as compared to tissue approximation in the absence of an implanted acellular tissue matrix. In various embodiments, the implanted acellular tissue matrix is secured to the surrounding tissue planes. The implanted acellular tissue matrix can be secured to the surrounding tissue planes using biodegradable sutures, positive external pressure to the tissue planes surrounding the implanted acellular tissue matrix, or through a reduced pressure therapy device.

In various embodiments, a kit is provided for use in treating a patient in need of tissue approximation, comprising an acellular tissue matrix and instructions for using the acellular tissue matrix by implanting the acellular tissue matrix between separated tissue planes. In some embodiments, the kit can further comprise a reduced pressure therapy device.

### DESCRIPTION OF THE DRAWINGS

Fig. 1A-B are photos of foam acellular tissue matrices prepared from human dermis according to certain embodiments of the methods disclosed herein. The foam in Fig. 1A has been further cross-linked with EDAC, while the foams in Figs. 1B and 1C have not.
Fig. 2 illustrates a reduced pressure therapy device for the delivery of reduced or negative pressure to an implanted acellular tissue matrix, according to certain embodiments disclosed herein.
Fig. 3 shows thermogram plots for porcine foam acellular tissue matrices that have undergone dehydrothermal treatment, as well as for porcine foam acellular tissue matrices that have not undergone dehydrothermal treatment, according to certain embodiments disclosed herein.
Fig. 4 shows tissue degradation over time for porcine foam acellular tissue matrices treated with Type I collagenase at 37°C, as prepared according to certain embodiments disclosed herein.
Fig. 5 illustrates a procedure according to certain embodiments for folding porcine foam acellular tissue matrices prior to subcutaneous implantation into immuno-competent rats.
Fig. 6A-D show explanted porcine foam acellular tissue matrices after a 4-week implantation in immuno-competent rats. Fig. 6A shows the gross appearance of explanted tissue foams. Fig. 6B shows the cross-section view of explanted tissue foams. Fig. 6C shows H&E staining of explanted tissue foams. Fig. 6D shows H&E staining of explanted tissue foams at a higher magnification. Arrows indicate re-vascularization.

### DESCRIPTION OF ILLUSTRATIVE EMBODIMENTS

Reference will now be made in detail to certain exemplary embodiments according to the present disclosure, certain examples of which are illustrated in the accompanying drawings.

In this application, the use of the singular includes the plural unless specifically stated otherwise. Also in this application, the use of "or" means "and/or" unless stated otherwise. Furthermore, the use of the term "including," as well as other forms, such as "includes" and "included," are not limiting. Any range described here will be understood to include the endpoints and all values between the endpoints.

The section headings are for organizational purposes only and are not to be construed as limiting the subject matter described. Disclosed are acellular tissue matrix compositions and methods of making and using the compositions. The compositions can be used to approximate tissue, thereby reducing space between tissue planes within which fluid or blood can collect. As used herein, "tissue approximation" means filling, sealing, or otherwise eliminating or reducing space between tissue planes. The term encompasses all compositions and methods that produce, promote, or otherwise enhance the natural or artificial sealing of tissue planes that have been separated by damage or disease.

Implanting acellular tissue matrices to reduce space between tissue planes can help prevent seroma/hematoma formation. Implanting the compositions can also promote native cell migration and proliferation from the surrounding tissue planes into the acellular tissue matrix. As a result, natural tissue adhesion can be enhanced as cells from separated tissue planes or other tissue (e.g., blood) enter and proliferate within the acellular tissue matrix. As used herein, the terms "native cells" and "native tissue" mean the cells or tissue present in the recipient organ or tissue prior to implantation of an acellular tissue matrix composition.

The materials and methods provided herein can be used to make a biocompatible tissue implant for use in approximating separated tissue. As used herein, a "biocompatible" composition is one that has the ability to support cellular activity necessary for tissue regeneration, repair, or treatment and does not elicit a substantial immune response that prevents such cellular activity. As used herein, a "substantial immune response" is one that prevents partial or complete tissue regeneration, repair, or treatment.

### Acellular tissue matrix compositions

Disclosed herein are acellular tissue matrix compositions for use in tissue approximation. As used herein, the terms "acellular tissue matrix" or "acellular tissue matrix composition" mean a composition comprising a decellularized tissue that is suitable for use to fill, seal, or otherwise eliminate or reduce space between tissue planes (i.e., suitable to use in tissue approximation) without eliciting a substantial immune response. In certain embodiments, the acellular tissue matrix is completely or substantially free of all cells present in the tissue prior to decellularization. As used herein, "substantially free of all cells" means that the acellular tissue matrix contains less than 20%, 10%, 5%, 1%, 0.1%, 0.01%, 0.001%, or 0.0001% (or any percentage in between) of the cells that normally grow within the acellular matrix of the tissue prior to decellularization.

In various embodiments, the acellular tissue matrix comprises at least a portion of an extracellular matrix from a decellularized tissue. The acellular tissue within the composition may be in either particulate or non-particulate form. In certain embodiments, the acellular tissue matrix is provided in a foam, film, or other porous form capable that is capable of providing a structural scaffold for native cell migration/proliferation and which is also suitable for implantation between separated tissue planes. Exemplary methods for preparing these acellular tissue matrices are described below.

The disclosed acellular tissue matrices can provide natural tissue scaffolds within which native cells and vasculature from surrounding tissue can migrate and proliferate. Cell migration from surrounding tissue into the acellular tissue matrix can result in enhanced natural sealing or adhering of separated tissue planes. Methods of using acellular tissue matrices to approximate tissue are described in further detail below.

The disclosed acellular tissue matrices can be derived from various organ or tissue sources. Acellular tissue matrices provide spongy, flexible materials after decellularization. In certain embodiments, this spongy tissue filler can be molded into desired shapes so as to fit into and/or fill the space between separated tissue planes. In some embodiments, the acellular tissue matrix retains substantial elasticity. For example, if the acellular tissue matrix is derived from dermis, then it can have at least the elasticity of non-decellularized dermis, if not increased elasticity. This elasticity allows an implanted acellular tissue matrix to flex, stretch, or compress within an implantation site, thereby helping to helping to maintain an intact collagen framework over a longer period of time.

In various embodiments, the acellular tissue matrices are capable of substantial stretching, torsion, or compression. For example, the tissue matrices may be compressed up to approximately 2/3 of their initial length or width. In still further embodiments, an acellular tissue matrix is capable of rapidly returning to its original dimensions after the release of compression.

The extracellular scaffold within an acellular tissue matrix may consist of collagen, elastin, or other fibers, as well as proteoglycans, polysaccharides and growth factors. An acellular tissue matrix may retain some or all the extracellular matrix components that are found naturally in a tissue prior to decellularization, or various undesirable components may be removed by chemical, enzymatic or genetic means. In general, the acellular matrix provides a structural network of fibers, proteoglycans, polysaccharides, and growth factors on which native tissue and vasculature can migrate, grow, and proliferate. The exact structural components of the extracellular matrix will depend on the tissue selected and the processes used to prepare the acellular tissue.
Acellular tissue matrix compositions for use in tissue approximation can be derived from various organ or tissue sources. Organs having a compact three dimensional shape may be selected, including organs such as lung, liver, bladder, muscle, fat, or dermis, which provide a spongy acellular matrix after decellularization. This spongy tissue can be further processed or molded into a desired shape to partially or completely fill the space between separated tissue layers. In some embodiments, the acellular tissue matrix compositions are provided as strips or balls. In other embodiments, the compositions are molded into any shapes or sizes that are suitable for use in approximating a particular tissue. Acellular tissue matrices can be derived from various animal sources. In certain embodiments, the acellular tissue is prepared from human cadaver, cow, horse, or pig tissue.

In certain embodiments, acellular tissue matrices are processed to lack certain undesirable antigens. For example, certain animal tissues contain alpha-galactose (a-gal) epitopes that are known to elicit reactions in humans. Therefore, acellular tissue matrices produced from animal tissues can be produced or processed to lack certain antigens, such as α-gal. In some embodiments, acellular tissue matrices lack substantially all α-gal moieties. Elimination of the α-gal epitopes from a natural tissue scaffold may diminish the immune response against an acellular tissue matrix composition. U. Galili et al., J. Biol. Chem. 263: 17755 (1988). Since non-primate mammals (e.g., pigs) produce α-gal epitopes, xenotransplantation of acellular tissue matrix material from these mammals into primates may result in rejection because of primate anti-Gal binding to the α-gal epitopes on the acellular tissue matrix. The binding results in the destruction of the acellular tissue matrices by complement fixation and by antibody-dependent cell cytotoxicity. U. Galili et al., Immunology Today 14: 480 (1993); M. Sandrin et al., Proc. Natl. Acad. Sci. USA 90: 11391 (1993); H. Good et al., Transplant. Proc. 24: 559 (1992); B. H. Collins et al., J. Immunol. 154: 5500 (1995).

As described in detail below, in various embodiments, acellular tissue matrices can be processed to remove antigens such as α-gal, e.g., by chemical or enzymatic treatment. Alternatively, the acellular tissue matrices can be produced from animals that have been genetically modified to lack those epitopes.

In various embodiments, acellular tissue matrices have reduced bioburden (i.e., a reduced number of microorganisms growing on the compositions). In some embodiments, acellular tissue matrices lack substantially all bioburden (i.e., the acellular tissue matrices are aseptic or sterile). As used herein, "substantially all bioburden" means acellular tissue matrices in which the concentration of growing microorganisms is less than 1%, 0.1%, 0.01%, 0.001%, or 0.0001% (or any percentage in between) of that growing on untreated acellular tissue matrices.

In certain embodiments, an acellular tissue matrix composition can further comprise one or more additional agents. In some embodiments, the additional agent is a non-biological (but biocompatible) mesh or other structure that provides a reinforced scaffold for native cell migration and proliferation. As used herein, a "mesh" is any composition comprising woven or interconnected strands of synthetic or biological fibers. Acellular tissue can overlay at least a portion of one surface of the mesh or scaffold, or vice versa.

In certain embodiments, an acellular tissue matrix composition can further comprise one or more additional agents, wherein the additional agents comprise stem cells or other viable cells that are histocompatible with the patient in which they are being implanted. In some embodiments, the histocompatible cells are mammalian cells. Such cells can promote native tissue migration, proliferation, and/or vascularization.

In some embodiments, the additional agent comprises at least one added growth or signaling factor (e.g., a cell growth factor, an angiogenic factor, a differentiation factor, a cytokine, a hormone, and/or a chemokine). These additional agents can promote native tissue migration, proliferation, and/or vascularization. In some embodiments, the growth or signaling factor is encoded by a nucleic acid sequence contained within an expression vector. Preferably, the expression vector is in one or more of the viable cells that can be included, optionally, along with the acellular tissue matrix. As used herein, the term "expression vector" refers to any nucleic acid construct that is capable of being taken up by a cell, contains a nucleic acid sequence encoding a desired protein, and contains the other necessary nucleic acid sequences (e.g. promoters, enhancers, termination codon, etc.) to ensure at least minimal expression of the desired protein by the cell.

The acellular tissue matrix compositions can further comprise additional synthetic or natural components, so long as these components promote natural cell migration and/or proliferation. For example, acellular tissue matrix compositions can contain synthetic meshes or other structures that enhance the acellular tissue matrix and thereby promote native cell in-growth. In some embodiments, synthetic meshes that surround or underlay an acellular tissue matrix can be used to reinforce the acellular matrix. Reinforcement may enable the acellular tissue matrix to provide a structural scaffold for native cell migration over a longer period of time.

### Methods of Production

The acellular tissue matrix compositions that are used to approximate tissue can be prepared according to various methods. In some embodiments, tissue is decellularized and, optionally, further processed to produce a foam, film, or other porous structure that can serve as an acellular tissue scaffold for native cell migration and proliferation.

An acellular tissue matrix can be prepared from any tissue that is suitable for decellularization and subsequent implantation for tissue approximation. Organs having a compact three dimensional shape may be used, including organs such as lung, liver, bladder, muscle, fat, or dermis, as they provide a spongy acellular matrix after decellularization. In certain exemplary embodiments, the acellular tissue matrix comprises ALLODERM® or STRATTICE™, which are acellular human dermal and porcine dermal products, respectively and are available from LifeCell Corporation (Branchburg, NJ).

The decellularized tissue in an acellular tissue matrix can be treated to render it aseptic or sterile. In some embodiments, the tissue can be further treated to remove contaminants, to sterilize the tissue, or to remove certain undesirable antigens.

Exemplary methods for decellularizing tissue and/or fragmenting decellularized tissue to produce particulate acellular tissue are disclosed in U.S. Patent 6,933,326 and U.S. Patent Application 2010/0272782. Particulate acellular tissue encompasses any generally spherical or irregular shaped tissue fragments having a longest dimension of less than about 5000 microns. Particulate acellular tissue can be made from any of the non-particulate acellular tissue disclosed herein. For example, particulate acellular tissue can be prepared by fragmenting non-particulate tissue before or after decellularization. For example, non-particulate tissue can be cut into strips, e.g., using a Zimmer mesher, then the strips can be cut to form small fragments of less than 5000 microns in size. The exemplary small fragments can be further homogenized using a homogenizer to produce small fragments of desired size. In some embodiments, the decellularized particulate tissue can be further processed to produce a foam, film, or other porous structure that provides a structural scaffold into which cells from surrounding native tissue can migrate and proliferate.

In certain embodiments, the acellular tissue matrix is prepared as a film. As used herein, the term "film" means any collagen sheet or coating containing an extracellular tissue matrix that is formed by swelling acellular tissue fragments and then drying the suspension in a mold to form a film of desired shape. In some embodiments, a film is prepared as disclosed in U.S. Application 2010/0272782.

In certain embodiments, the acellular tissue matrix is prepared as a porous foam. As used herein, the term "foam" means any porous collagen material containing an extracellular tissue matrix that is formed by forming a suspension of acellular tissue fragments and then freeze-drying the suspension in a mold to form a foam of desired shape. In some embodiments, a foam is prepared as disclosed in U.S. Application 2010/0272782.

In some embodiments, a particulate acellular tissue matrix composition is made by micronizing, cryofracturing, homogenizing (e.g. via blender, bead mill, ultrasonic homogenizer, or any other homogenizer) or otherwise fragmenting decellularized tissue. The fragmented acellular tissue can be in the form of particles, fibers, threads, or other fragments. Exemplary methods of fragmenting decellularized tissue are disclosed in U.S. Patent 6,933,326 and U.S. Patent Application 2010/0272782. In some embodiments, the fragmented acellular tissue can then be placed in an acidic solution to create a homogenous suspension of swollen acellular tissue. Prior to swelling the fragmented tissue, the acellular tissue matrix can be washed to remove any residual cryoprotectants or other contaminants. Solutions used for washing can be any physiologically-compatible solution. Examples of suitable wash solutions include distilled water, phosphate buffered saline (PBS), or any other biocompatible saline solution.

The acid used to swell the acellular tissue fragments can be any acid that will maintain the fragments as a homogenous suspension and does not result in substantial, irreversible denaturation of the collagen fibers in the tissue fragments. As used herein, the term "homogenous suspension" is a suspension in which the acellular tissue fragments are not larger than 3mm in diameter (e.g. not more than 3.0mm., 2.5mm. 2.0mm. 1.5mm, 1000 µm, 900 µm, 800 µm, 700 µm, 600 µm, or 500 µm in diameter, or any value in between), and the fragments are distributed in a liquid medium. In some embodiments, the acid solution used to swell the acellular tissue fragments will have a pH approximately at or below 3.0 (e.g., a pH at or below 3.2, 3.15, 3.05, 3.0, 2.95, 2.85, 2.8, 2.75, or any value in between). Examples of useful acids include acetic acid, ascorbic acid, boric acid, carbonic acid, citric acid, hydrochloric acid, lactic acid, peracetic acid, phosphoric acid, sulfuric acid, tannic acid, and trichloroacetic acid. Combinations of two or more acids can also be used.

The specific concentration of acid will depend on the strength of the acid used. For example, if acetic acid is selected, the acid could be used, in certain embodiments, at a concentration between approximately 25mM and 250mM (i.e., approximately 25, 50, 75, 100, 125, 150, 175, 200, 225, or 250mM, or any concentration in between). Another exemplary acid is hydrochloric acid (HCI). HCI can be used, in certain embodiments, at a concentration between approximately 25mM and 200mM (e.g., approximately 25, 50, 75, 100, 125, 150, 175, or 200 mM, or any value in between). The term "approximately," as used here, encompasses any concentration that varies from the stated concentration by 10% or less (i.e., a concentration of 50mM encompasses all concentrations between 45mM and 55mM).

The acellular tissue fragments can be swollen in acid for any period of time required to produce a homogenous suspension of acellular tissue fragments. For example, swelling can occur for approximately 0.5, 1.0, 1.5, 2.0, 2.5, 3.0, 3.5, 4.0, 4.5, 5.0, 5.5, 6.0, 6.5, 7.0, 7.5, 8.0, 9.0, 10, 11, 12, 14, 16, 18, 22, 24, 36, or 48 hours (or any length of time in between). The term "approximately" encompasses all times that vary by up to 0.2 hours above or below the stated time (i.e., a 2.0 hour swelling time encompasses all swelling times ranging from 1.8 hours to 2.2 hours).

In certain embodiments, swelling is achieved by contacting acellular tissue fragments with an acid solution at a temperature slightly above room temperature (e.g., at an approximate temperature of 28, 29, 30, 31, 32, 33, 34, 35, 36, 37, 38, 39, 40, 41, 42, 43, 44, or 45 degrees Celsius, or any temperature in between). The term "approximately" includes any temperature that varies by 0.5°C (i.e., a temperature of 30.0°C includes all temperatures between 29.5°C and 30.5°C).

The type of acid, concentration of acid, length of exposure to acid, and temperature used during swelling can be adjusted to achieve optimal swelling of the acellular tissue fragments while avoiding collagen denaturation. For example, acellular tissue from different animal sources may require different swelling conditions in order to achieve optimal swelling.

The final concentration of acellular tissue fragments in the homogenous solution can be any concentration where swelling occurs uniformly and results in a homogenous suspension. For example, useful concentrations (w/v) of acellular tissue matrix fragments in acidic solution can range from about 0.1% to 4.0% (e.g., 0.1%, 0.2%, 0.3%, 0.4%, 0.5%, 1.0%, 1.5%, 2.0%, 2.5%, 3.0%, 3.5%, or 4.0%, or any percentage in between). As used here, the term "about" encompasses any percentage that varies from the indicated percentage by up to 0.5% (i.e., a 2.0% solution encompasses all solutions having 1.5% to 2.5% acellular tissue fragments, measured w/v).

In certain embodiments, once the acellular tissue fragments have been swollen in an acid solution, the homogenous solution is then applied to a mold of desired shape and allowed to dry to produce a porous film. Drying can be by any method known in the art that will result in the retention of desired biological and physiological properties of the acellular tissue, including, e.g., the structure of collagen fibers and the extracellular framework. Exemplary drying methods include air drying or drying under inert gas (e.g., nitrogen or argon). The drying temperature may be ambient temperature, e.g. approximately 25°C. As used here, "approximately" means any temperature within a 3°C range of the stated 25°C drying temperature (i.e., 22.0°C, 22.5°C, 23.0°C, 23.5°C, 24.0°C, 24.5°C, 25.0°C, 25.5°C, 26.0°C, 26.5°C, 27.0°C, 27.5°C, 28.0°C, or any temperature in between). Alternatively, drying can be done at a temperature that is moderately above room temperature, e.g., at a temperature from approximately 25°C to 45°C. For example, drying can be done at approximately 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, or 45°C, or any temperature in between. As used here, "approximately" means any temperature within 0.5°C of the stated temperature (i.e., a temperature of 40°C includes any temperature between 39.5°C and 40.5°C). Drying may employ convection, conduction, or any other method that will transfer heat through direct or indirect contact in order to dry the homogenous suspension in the mold.

In alternative embodiments, the homogenous solution described above can be poured into a mold and freeze dried to produce a foam composition. Exemplary methods are disclosed in U.S. Patent Application 2010/0272782 and U.S. Patents 5,364,756; 5,780295; and 6,194,136. Freeze drying involves the removal of water and/or other solvents from a frozen product and may be accomplished by a variety of methods, including the manifold, batch, or bulk methods. Conditions for freeze drying, including timing, cooling velocity, and final temperature can vary depending on the freeze drying technique employed.

In certain embodiments, acellular foam compositions can be prepared by first chopping or cutting decellularized tissue into small fragments and then homogenizing the fragments. As used here, "small" means approximately 3mm or less in diameter (e.g. not more than 3.0mm., 2.5mm. 2.0mm. 1.5mm, 1000 µm, 900 µm, 800 µm, 700 µm, 600 µm, or 500 µm in diameter, or any value in between). "Approximately" in this context means any diameter within 0.5mm of the stated diameter (i.e., a 1.0mm fragment includes fragments having a diameter between 0.5mm and 1.5mm). The fragments can be homogenized by, e.g. using a blender, bead mill, ultrasonic homogenizer, or any other homogenizer. In some embodiments, homogenized tissue can be washed using any suitable wash solution (e.g. distilled water or PBS) and then suspended in an acid solution as described above. Acid-tissue suspensions can then be placed in a mold of desired shape and freeze dried (e.g., using Tyvek® freeze drying bags). Conditions for freeze drying, including timing, cooling velocity, and temperature can vary depending on the freeze drying technique employed.

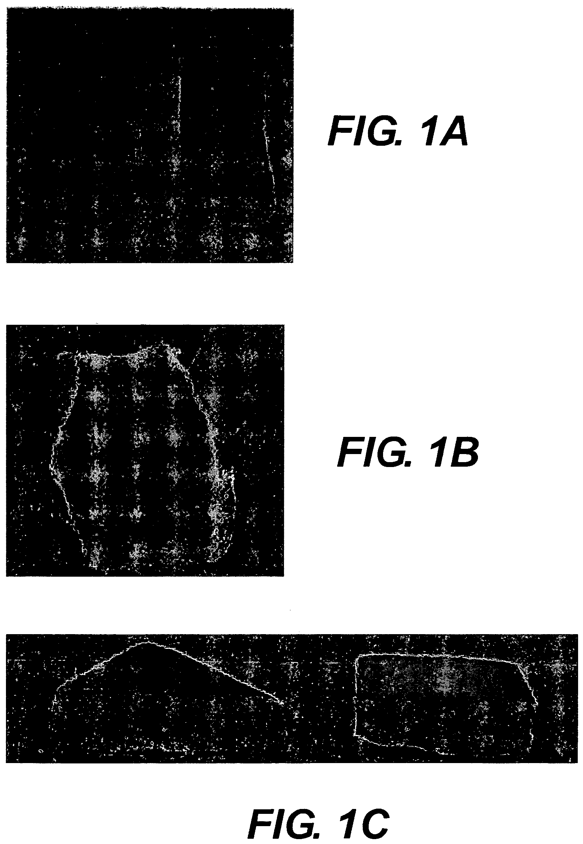

In certain embodiments, the foam and film compositions described above can be further stabilized via chemical cross-linking of the collagen molecules to each other or to various other biological or synthetic materials (e.g., crosslinking to synthetic meshes that provide enhanced structural support for acellular tissue). Cross-linking agents may include glutaraldehyde, carbodiimides (including 1-ethyl-3-[3-dimethylaminopropyl]carbodiimide hydrochloride (EDAC)), bisdiazobenzidine, N-maleimidobenzoyl-N-hydroxysuccinimde ester, and N-hydroxysulfosuccinimide (NHS). It is also possible to use combinations of cross-linking agents. Other potential cross-linking agents and methods of cross-linking are described in U.S. Patent Application 2010/0272782. **Fig. 1** shows foam acellular tissue matrices prepared from human dermis (the foam in Fig. 1A is cross-linked using EDAC).

In some embodiments, cross-linking is achieved via dehydrothermal treatment (e.g., curing an acellular tissue matrix in a heat oven under vacuum conditions). In certain embodiments, dehydrothermal treatment can be conducted at a temperature between approximately 80°C and 120°C (e.g., approximately 80°C, 85°C, 90°C, 95°C, 100°C, 105°C, 110°C, 115°C, or 120°C, or any temperature in between). The term "approximately" in this context means any temperature within 2°C of the stated temperature (i.e., a stated temperature of 100°C encompasses all temperatures between 98°C and 102°C).

In certain embodiments, cross-linking can be carried out by hydrating a film or foam composition in a solution containing cross-linking agent(s). Alternatively, cross-linking reagents that are active at acidic pH can be added directly to the acidic solution containing swollen acellular tissue fragments. The duration of the cross-linking reaction may vary depending on the cross-linking reagent used, the concentration of cross-linker, the type of acellular tissue, the reaction temperature, and the desired degree of cross-linking.

In some embodiments, the acellular tissue matrix compositions for use in tissue approximation can be treated to reduce bioburden (i.e., to reduce the number of microorganisms growing on the composition). In some embodiments, the compositions are treated such that they lack substantially all bioburden (i.e., the compositions are aseptic or sterile). As used herein, "substantially all bioburden" means that the concentration of microorganisms growing on the composition is less than 1%, 0.1%, 0.01%, 0.001%, or 0.0001% of that growing on untreated compositions, or any percentage in between. Suitable bioburden reduction methods are known to one of skill in the art, and may include irradiation. Irradiation may reduce or substantially eliminate bioburden. In some embodiments, an absorbed dose of 15-17kGy of E-beam radiation is delivered in order to reduce or substantially eliminate bioburden. Other irradiation methods are disclosed in U.S. Application 2010/0272782.

In further embodiments, acellular tissue compositions are treated with alpha-galactosidase to remove alpha-galactose (α-gal) moieties. In some embodiments, to enzymatically remove α-gal epitopes, after washing tissue thoroughly with saline, the tissue may be subjected to one or more enzymatic treatments to remove α-gal antigens, if present in the sample. In some embodiments, the tissue may be treated with an α-galactosidase enzyme to eliminate α-gal epitopes. In further embodiments, the tissue is treated with α-galactosidase at a concentration of 0.2 U/ml prepared in 100 mM phosphate buffered saline at pH 6.0. In other embodiments, the concentration of α-galactosidase is reduced to 0.1 U/ml or increased to 0.3 or 0.4 U/ml (or any value in between). In other embodiments, any suitable enzyme concentration and buffer can be used as long as sufficient antigen removal is achieved. In addition, certain exemplary methods of processing tissues to reduce or remove alpha-1,3-galactose moieties are described in Xu et al., Tissue Engineering, Vol. 15, 1-13 (2009).

Alternatively, in certain embodiments, animals that have been genetically modified to lack one or more antigenic epitopes may be selected as the tissue source for an acellular tissue matrix composition. For example, animals (e.g., pigs) that have been genetically engineered to lack the terminal α-galactose moiety can be selected as the tissue source. For descriptions of appropriate animals and methods of producing transgenic animals for xenotransplantation, see U.S. Patent Application Serial Number 10/896,594 and U.S. Patent No. 6,166,288.

### Methods of Use

A major challenge following surgery or other tissue disease/trauma is the formation of seromas or hematomas within the space created between tissue planes. Current therapies involve the use of drains to remove fluid from the space between tissue planes using passive drainage. Such drainage may necessitate extended medical care while potentially failing to eliminate the space between the tissue planes. Proposed alternatives involving glues to artificially close the space between tissues are still being developed. Thus, various alternative solutions for tissue approximation are needed. In certain embodiments, the acellular tissue matrices disclosed above are used to fill space between tissue planes and/or to promote approximation of tissue planes.

As used herein, the term "tissue plane" means any layer of tissue that has been separated from another tissue layer to which it is normally attached, resulting in the formation of a space between the tissue layers. The separation may occur naturally, as through disease that leads to the dissociation of tissue layers, or may occur artificially through trauma or surgical intervention that results in tissue separation. As used herein, the term "space" means the region between two or more separated tissue planes that results from disease, trauma, or surgical intervention. The space may be empty or filled with fluid or other material. It will be understood that the space can be formed by the separation of natural tissue planes or natural tissue boundaries. For example, the space may form at the boundary between two different tissue types (e.g., between a muscle and associated fascia, between a membrane and associated viscera, or between a tendon and surrounding sheath, among other examples). Alternatively, the space can form within a single tissue (e.g., by surgical dissection within fat to form separated fat tissue planes). Further, it will be understood that "tissue planes," as used herein, can be planar, but may also be curved or contoured in accordance with natural anatomy, or as a result of disease, trauma, or surgical intervention.

In certain embodiments, following the creation of space between tissue planes as a result of disease, trauma, or surgical intervention, an acellular tissue matrix composition (as described above) is placed between the separated tissue planes. The acellular tissue matrix can be secured to the separated tissue planes using any known method that results in the temporary or permanent physical association of the acellular tissue matrix with the proximate tissue planes. For example, biodegradable sutures can be used to physically secure the acellular tissue matrix to the separated tissue planes.
Alternatively, external positive pressure (e.g., a dressing or binding around the separated tissue planes) can be applied to compress the separated tissue planes and thereby maintain the tissue planes in contact with the implanted acellular tissue matrix.

In certain embodiments, internal negative pressure can be applied within the acellular tissue matrix to secure the implant to surrounding tissue planes. Internal negative pressure can pull the separated tissue planes towards the implanted acellular tissue matrix and thereby maintain contact between the implant and the surrounding tissue planes. In some embodiments, internal negative pressure can also be used to actively remove fluid or blood from the space between separated tissue layers, thereby reducing, treating, or preventing seroma or hematoma formation. In certain embodiments, negative pressure can also serve to draw cells from surrounding tissue into the implanted acellular tissue matrix, increasing the rate at which native cells migrate into the tissue matrix and enhancing the speed and/or overall effectiveness of tissue approximation.

In certain exemplary embodiments, internal negative pressure is delivered to the acellular tissue matrix by a reduced pressure therapy device. The reduced pressure therapy device can include a pump fluidly connected, e.g., through a fluid passage or tubing to the acellular tissue matrix, and which delivers reduced or negative pressure to the acellular tissue matrix. A variety of reduced pressure therapy devices can be used. For example, suitable reduced pressure therapy devices include V.A.C.® therapy devices produced by KCI (San Antonio, Texas).

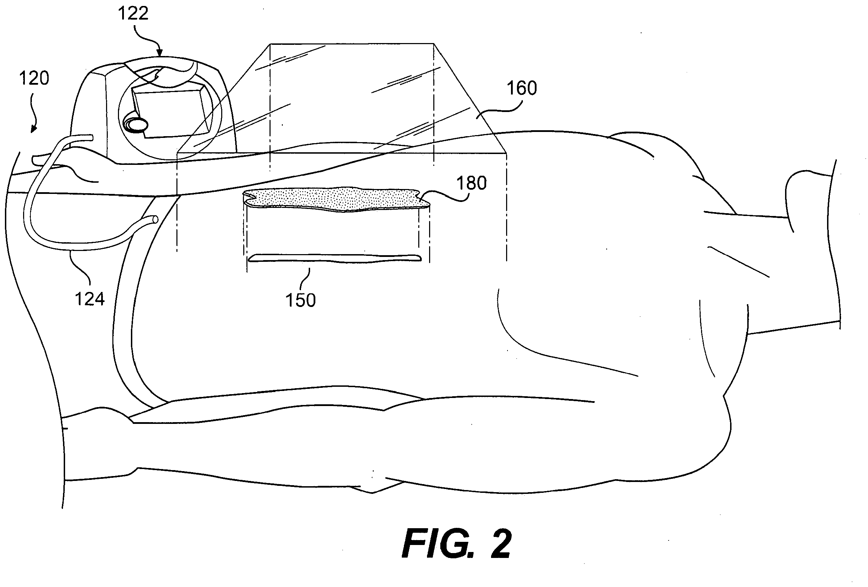

In some embodiments, reduced pressure therapy devices can include a vacuum pump, similar to the pump 122 shown in Fig. 2. The pump 122 can be fluidly connected, e.g., through a fluid passage or tubing 124, to an acellular tissue matrix 180 such that negative pressure is distributed within the implanted acellular tissue matrix. The porous nature of the acellular tissue matrix 180 allows it to channel negative pressure and/or to remove fluid from the implant site 150. Such devices may also include a flexible sheet, drape, or dressing 160 to cover the site where the acellular tissue matrix is implanted 150 and at least partially seal the site so that reduced pressure therapy can be provided at the site of implantation150 by the reduced pressure device 120.

In certain embodiments of the reduced pressure device, an optional porous manifold can be used to more evenly distribute negative pressure within the acellular tissue matrix. In some embodiments, the manifold is attached to the end of the fluid passage or tubing and is placed within or near the acellular tissue matrix. In some embodiments, the manifold is a porous polymeric structure capable of channeling negative pressure evenly throughout an acellular tissue matrix or throughout a tissue implant site. The manifold can be produced from a variety of suitable materials. For example, a number of different materials are available for use as manifolds with the above-noted V.A.C.® treatment systems. Such materials can include porous open-cell polymeric foam structures, such as open-cell polyetherurethanes (such as VAC® GRANUFOAM® Dressing, KCl, San Antonio, Texas). In various embodiments, the specific manifold used may be selected based on the particular site where the acellular tissue matrix is implanted.

In some embodiments, the reduced pressure system will include an adhesive that can facilitate attachment of the flexible sheet 160 to tissue or to other components of the negative pressure system. As used here, "adhesive" refers to any substance that causes the surfaces of two objects to be attached to one another. In various embodiments, suitable adhesives can include a variety of different cements, glues, resins, or other materials that can facilitate attachment of the flexible sheet 160 to tissue or to other components of the negative pressure system. In some embodiments, the adhesive can include a pressure-sensitive acrylic adhesive. In various embodiments, the adhesives can be applied directly to the structures to be joined, or the adhesives may be applied to tape, or with other supporting substrate materials.

In some embodiments, the adhesive can be applied to a surface of the flexible sheet 160 to attach the sheet to skin or other tissue surrounding the site of an implanted acellular tissue matrix. In some embodiments, the adhesive will be applied to the surface of the sheet 160 and packaged and/or distributed with the sheet 160. In some embodiments, the adhesive is applied to a surface of the sheet 160 and covered by a non-adhesive material that can be removed to expose the adhesive for use. In certain embodiments, the adhesive can be supplied as a separate component (e.g., in a container or on a tape) that is applied to the sheet 160 to attach the sheet 160 to tissue.

In some embodiments, a method is provided for applying reduced pressure to an implanted acellular tissue matrix in order to enhance tissue approximation. In certain embodiments, reduced or negative pressure is delivered to an implanted acellular tissue matrix using a reduced pressure therapy device such as the device shown in Fig. 2. In certain embodiments, the acellular tissue matrix 180 is placed between separated tissue planes. Next, the acellular tissue matrix 180 is fluidly coupled to the reduced pressure device 122 by a fluid passage or tubing 124. Then, after the negative pressure system 120 is positioned, the flexible sheet 160 is attached over the implant site 150, with the edges of the sheet 160 overlying the margins of the implant site 150 by a sufficient distance to allow a seal to be formed. It will be understood that the above example is only one embodiment of the reduced pressure system and variations are contemplated within the present disclosure. For example, in some embodiments where the separated tissues are internal to the body or where the separated tissues are sealed using sutures (or other suitable techniques), a drape may not be necessary in order to deliver negative pressure. Similarly, in certain embodiments where a more even distribution of negative pressure is desired, a porous polymeric manifold can be used to deliver negative pressure in or near the implanted acellular tissue matrix.

In certain embodiments, an acellular tissue matrix is implanted to approximate tissue that is separated due to surgical intervention. Surgical separation of tissue planes can occur within various tissues, and an acellular tissue matrix can be used to approximate any such tissues. In certain embodiments, the tissue being treated is breast tissue, where space (at risk for seroma/hematoma formation) is created following various types of breast surgery (e.g., cosmetic breast surgeries or mastectomies). In other embodiments, the seroma or hematoma being treated results from plastic or cosmetic surgery (e.g., facelift). In still other embodiments, the seroma or hematoma results from abdominal surgery.

In various embodiments, the implanted acellular tissue matrix fills at least a portion of the space between tissue planes in which seromas or hematomas could form. In some embodiments, the implanted acellular tissue matrix is amenable to the migration and/or proliferation of native cells within the acellular matrix. Migration and proliferation of native cells from two or more tissue planes surrounding the implant can result in enhanced natural approximation of separated tissue planes. In some embodiments, the migration and proliferation of native cells within the acellular tissue matrix may lead to faster tissue approximation of the separated tissue planes than could be achieved by the same tissue planes in the absence of an implanted acellular tissue matrix. In certain embodiments, negative pressure therapy is applied internally to the implanted acellular tissue matrix, thereby enhancing the rate or overall effectiveness of tissue approximation by increasing the speed and/or overall level of native cell migration into the implanted acellular tissue matrix. Negative pressure therapy can also help drain fluid from the space between separated tissue planes, thereby helping to treat or prevent seroma/hematoma formation.

The methods of using acellular tissue matrix compositions to approximate tissue (described above) can be used to treat a patient wherein damage or disease has resulted in the separation of tissue planes. In certain embodiments, implantation of acellular tissue matrices, as described above, can be used to fill space between separated tissue planes and thereby help prevent seroma or hematoma formation. In some embodiments, the implanted acellular tissue matrix can also provide a structural scaffold into which native cells from the surrounding tissue migrate and proliferate, thereby encouraging faster approximation of separated tissue planes, as compared to sealing in the absence of an implanted acellular tissue matrix.

Also disclosed herein are kits for use in treating a patient in need of tissue approximation, comprising an acellular tissue matrix composition (as described above) and instructions for using the acellular tissue matrix composition by implanting it between separated tissue planes. The kit can further include a reduced pressure therapy system, as described above.

### EXAMPLES

The following examples serve to illustrate, and in no way limit, the present disclosure.

### Example 1: Preparation of Foam Acellular Tissue Matrices

55g - 60g of decellularized human dermis was washed with saline, chopped into small pieces (1-2mm), and homogenized in 500mL saline into small tissue fibers with a blender (Model 7011HS, Waring Commercial, Torrington, CT). Tissue fibers were washed with distilled water twice using centrifugation and then were suspended in 150 ml distilled water (∼7% tissue mass, w/v). An equal volume of 0.2% (w/v) peracetic acid solution (neutralized to a pH 7.0 with NaOH) was added to sterilize the tissue fibers for 2 hours. Peracetic acid residue was washed with sterile water twice.

The tissue fiber suspensions were cast in weighing boats that were sealed in Tyvek bags for freeze-drying. The freeze-drying conditions were: cooling from room temperature to 0°C at 2°C/min; cooling from 0°C to -50°C at 0.5°C/min; primary drying at a shelf temperature of -15°C for 36 hours under a chamber pressure of 6.7 Pa;
secondary drying at 20°C for 6 hours. Freeze-dried human dermis foams had a density of about 4% (w/v). Dried tissue foams had a moisture of ∼1.5%, and a thermal denaturation temperature about 125°C to 140°C.

Cross-linking treatments were applied to some acellular tissue matrices to further stabilize tissue foams, including the use of 0.1% glutaraldehyde, 20mM EDAC + 10 mM NHS, and dehydrothermal treatment.

For cross-linking with glutaraldehyde: glutaraldehyde was diluted with distilled water to a final concentration of 0.1% (w/v). Freeze-dried tissue foams were directly rehydrated in 0.1% glutaraldehyde solution at 4°C for 24 hours. After cross-linking, tissue foams were rinsed with distilled water and freeze dried again. The freeze drying conditions were: cooling from room temperature to -35°C at 0.2°C/min and drying at a shelf temperature -10°C for 40 hours.

For cross-linking with EDAC: 5 µM lysine, 20 mM EDAC (1-Ethyl-3-[3-dimethylaminopropyl] carbodiimide hydrochloride) and 10 mM NHS (N-hydroxysulfosuccinimide) were dissolved in 50 mM MES buffer (pH 5.5). Freeze-dried tissue foams were directly rehydrated in EDAC solution at 4°C for 24 hours. After cross-linking, tissue foams were rinsed with distilled water and freeze dried. The freeze drying conditions were: cooling from room temperature to -35°C at 0.2°C/min and drying at a shelf temperature -10°C for 40 hours.

For dehydrothermal treatment: tissue foams were loaded into a vacuum oven. The oven chamber was depressurized and heated to 100°C - 105°C. Treatment was maintained for 20 hours.

### Example 2: Evaluation of Porcine Foam Acellular Tissue Matrices

### Preparation of porcine foam acellular tissue matrices

Porcine foam acellular tissue matrices were made from commercially available STRATTICE™ reconstructive tissue matrix (LifeCell Corporation, Branchburg, New Jersey). All process steps were carried out under aseptic conditions. STRATTICE™ sheets were washed in sterile water overnight. Washed tissue sheets were rolled into a cylindrical shape, packaged aseptically, and frozen at -80°C. Frozen tissue rolls were grated using a household cheese grater to make tissue fibers. Grated tissue fibers were suspended in sterile water at ∼8 mL water per gram tissue, and the suspension was blended at 4000 rpm with a household blender (Retsch, PA). Blending was done in one minute intervals, followed by a 5 minute wait, for an overall 5 minute blending time. The suspension was then cast in square plastic Petri dishes (80 cm²), each containing 25-30 ml of suspension. Petri dishes were cooled from room temperature to -30°C in 60 minutes to freeze the suspension, and freeze-dried at a shelf temperature of 20°C for 36 hours at a chamber pressure of 13.3 Pa.

### Dehydrothermal treatment

Freeze-dried porcine foam acellular tissue matrices were weak and fragile. To stabilize and strengthen freeze-dried tissue foams, the tissue foams were subjected to dehydrothermal treatment (DHT). Tissue foams in sterile Tyvek bags were placed into a vacuum chamber: the pressure of the chamber was reduced to 13.5 kPa and the temperature of the chamber was increased to 100 ± 5°C for a 24-hour dehydrothermal treatment.

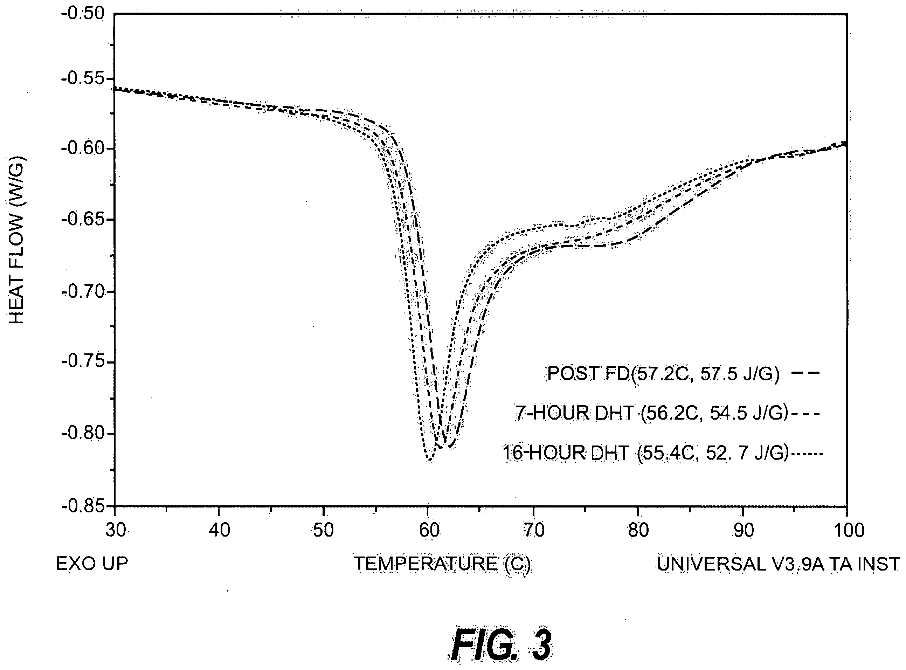

Thermal stability of collagen fibers in tissue foams was measured with differential scanning calorimetry. **Fig. 3** shows thermograms of porcine tissue foams with and without the dehydrothermal treatment (DHT). Samples of tissue foams (4 to 5 mg per sample) were rehydrated in 0.9% saline at 4°C overnight. Rehydrated samples were blot-dried to remove surface water, and hermetically sealed in crucibles. Collagen denaturation was measured at a scan rate of 3°C/min from 2 to 110°C. The denaturation temperature and enthalpy of tissue foams were slightly decreased as the duration of dehydrothermal treatment increased. But, the denaturation temperature of tissue foams remained approximately 15°C higher than the human body temperature.

The increase of advanced glycation end-products (i.e., Maillard products) in foam acellular tissue matrices was measured after the solubilization of foam samples with proteinase K at 10 mg/ml in 10 mM Tris-HCI (pH 7.5) containing 2 mM CaCl₂. The content of Maillard products was measured by fluorescence at an excitation wavelength of 360 ± 20 nm and emission wavelength of 440 ± 5 nm, with pentosidine as a standard. The content of Maillard products in freeze-dried foams that had not been subjected to DHT was 152 ± 8 µmol/g of foam material. After DHT, content of Maillard products increased to 194 ± 7 µmol/g of foam material, indicating some cross-linking between collagen fibers during DHT.

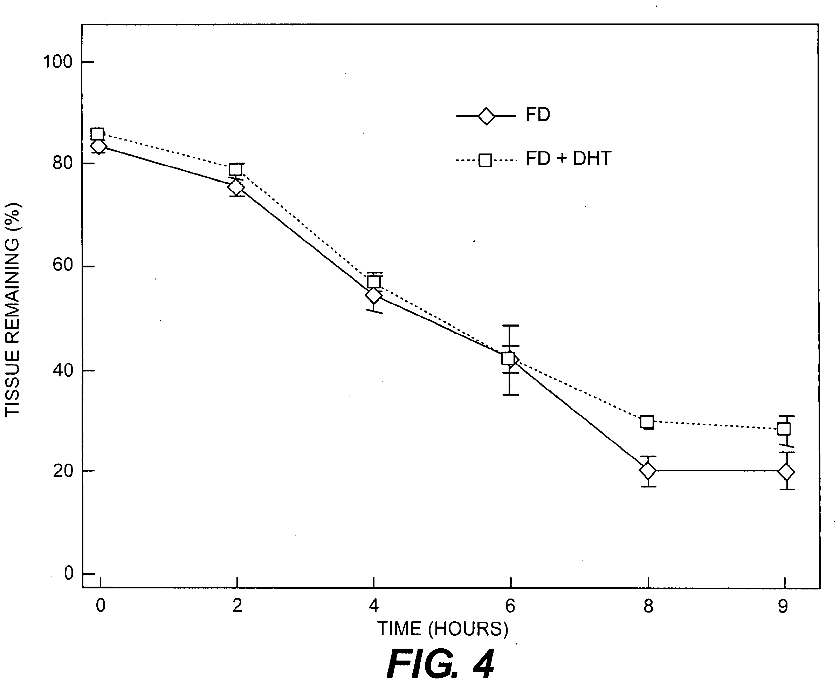

Since Type I collagen is a major component of foam acellular tissue matrices, change in the foam tissue matrix's susceptibility to collagenase after DHT was investigated. 20 to 30 mg samples of foam material were rehydrated in 1.5 mL of Tris-HCI buffer at 2 to 8°C overnight. An aliquot of 60 µL of collagenase stock solution (10 mg/mL) was added for each sample and enzyme digestion was performed at 37°C for up to 9 hours. The percentage of tissue remaining after collagenase digestion for various lengths of time was significantly higher after DHT. See **Fig. 4****.** This data is consistent with the measurement of increased Maillard products after DHT, suggesting increased cross-linking during DHT.

### In vivo responses with immune-competent rats

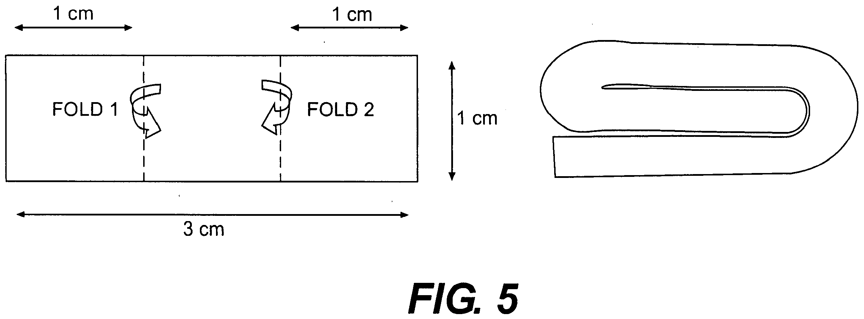

Porcine foam acellular tissue matrices (6 to 8% w/v) were evaluated by subcutaneous implantation into immune-competent rats. Four pieces of tissue foam (each having dimensions of 1 cm x 3 cm, 2 mm thick) were rehydrated in 0.9% saline, and each piece was folded to form a 3-layer thick foam implant (having dimensions 1 cm x 1 cm, ∼6 mm thick), as shown in **Fig. 5****.**

Folded tissue foams were implanted into subcutaneous pockets on the backs of rats. The pockets were formed by blunt dissection to separate tissue layers. After placement of folded tissue foams, the skin was closed with 4-0 non-absorbable sutures. Four (4) weeks following implantation, animals were euthanized, and the implanted tissue foams were recovered for the assessment of biological responses.

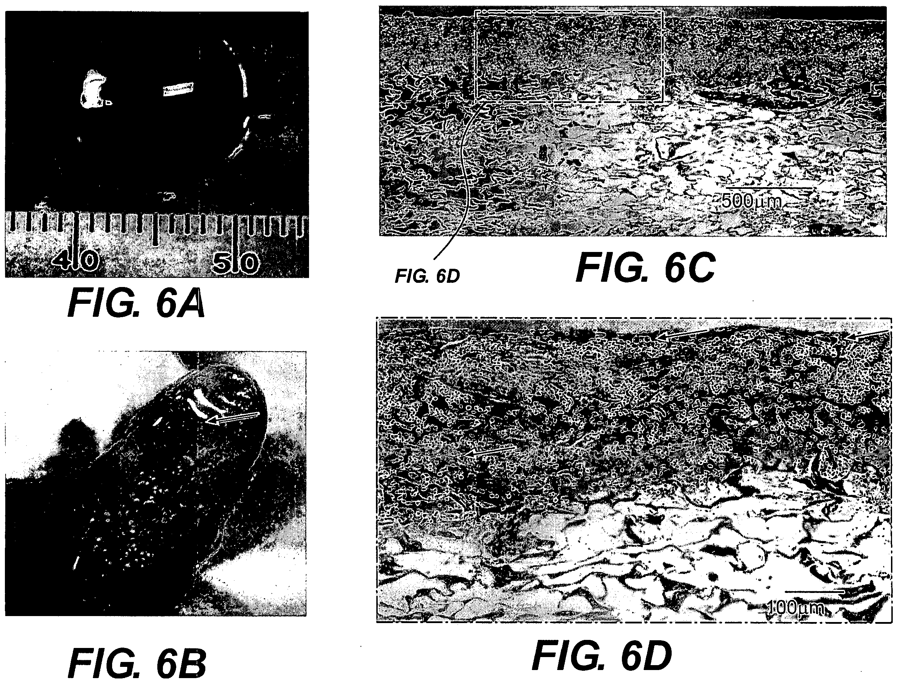

Gross assessment of explanted porcine foam acellular tissue matrices showed that tissue foams were well integrated within the animal tissue and had started to re-vascularize (arrows in Fig. 6B and 6D). See **Fig. 6****.** There was no significant decrease in the volume of implanted foam material. Host cells had already repopulated the outer layer of tissue foams, and formed new extracellular tissue matrix material. The interior of tissue foams remained porous without collapse.

The preceding examples are intended to illustrate and in no way limit the present disclosure. Other embodiments of the disclosed devices and methods will be apparent to those skilled in the art from consideration of the specification and practice of the devices and methods disclosed herein.

## Claims

1. An acellular tissue matrix comprising an extracellular matrix of a decellularized tissue for use in a method to prevent the formation of a seroma or hematoma by approximating separated tissue by implanting the acellular tissue matrix into a space between two or more separated tissue planes. wherein the implanted acellular tissue matrix comprises a foam capable of supporting migration and proliferation of cells from surrounding tissue following implantation.

2. The acellular tissue matrix for the use of claim 1 , wherein the implanted acellular tissue matrix further comprises one or more viable cells that are histocompatible with the patient in which they are being implanted.

3. The acellular tissue matrix for the use of claim 2, wherein the one or more viable cells are stem cells.

4. The acellular tissue matrix for the use of claim 1, wherein the implanted acellular tissue matrix is secured to the surrounding tissue planes using biodegradable sutures.

5. The acellular tissue matrix for the use of claim 1, wherein the implanted acellular tissue matrix is secured to the surrounding tissue planes by applying positive external pressure to the tissue planes surrounding the implanted acellular tissue matrix.

6. The acellular tissue matrix for the use of claim 5, wherein the positive external pressure comprises a dressing or binding around the tissue planes.

7. The acellular tissue matrix for the use of claim 1, further comprising a reduced pressure therapy device.

8. The acellular tissue matrix for the use of claim 7, wherein the reduced pressure therapy device comprises a negative pressure source that is fluidly connected by a fluid passage or tubing to the acellular tissue matrix and wherein the reduced pressure therapy device delivers negative internal pressure to the acellular tissue matrix.

9. The acellular tissue matrix for the use of claim 8, wherein the acellular tissue matrix is secured to surrounding tissue planes by the negative internal pressure.

10. The acellular tissue matrix for the use of claim 8, further comprising a porous manifold that is placed in or near the implanted acellular tissue matrix, is connected to the negative pressure source by the fluid passage or tubing, and distributes negative pressure within or near the implanted acellular tissue matrix.

11. The acellular tissue matrix for the use of claim 8, wherein the negative pressure source is a pump.

12. The acellular tissue matrix for the use of claim 8, further comprising a drape to seal a site where an acellular tissue matrix has been implanted.

13. A kit for use in treating a patient in need of tissue approximation to prevent the formation of a seroma or hematoma, comprising the acellular tissue matrix of claim 1 and instructions for using the acellular tissue matrix by implanting the acellular tissue matrix between separated tissue planes.

14. The kit of claim 13, further comprising a reduced pressure therapy device.

## Patentansprüche

1. Azelluläre Gewebematrix, umfassend eine extrazelluläre Matrix eines dezellularisierten Gewebes, zur Verwendung in einem Verfahren zur Verhinderung der Bildung eines Seroms oder Hämatoms durch Annähern von getrenntem Gewebe durch Implantieren der azellulären Gewebematrix in einen Raum zwischen zwei oder mehr getrennten Gewebeebenen, wobei die implantierte azelluläre Gewebematrix einen Schaum umfasst, der eine Migration und eine Proliferation von Zellen aus umgebenden Gewebe nach der Implantation unterstützen kann.

2. Azelluläre Gewebematrix zur Verwendung nach Anspruch 1, wobei die implantierte azelluläre Gewebematrix weiterhin eine oder mehrere lebensfähige Zellen umfasst, die mit dem Patienten, in den sie implantiert werden, histokompatibel sind.

3. Azelluläre Gewebematrix zur Verwendung nach Anspruch 2, wobei die eine oder die mehreren lebensfähigen Zellen Stammzellen sind.

4. Azelluläre Gewebematrix zur Verwendung nach Anspruch 1, wobei die implantierte azelluläre Gewebematrix an den umgebenden Gewebeebenen unter Verwendung von biologisch abbaubaren Nähten gesichert wird.

5. Azelluläre Gewebematrix zur Verwendung nach Anspruch 1, wobei die implantierte azelluläre Gewebematrix an den umgebenden Gewebeebenen durch Anwenden eines externen Überdrucks auf die Gewebeebenen, die die implantierte azelluläre Gewebematrix umgeben, gesichert wird.

6. Azelluläre Gewebematrix zur Verwendung nach Anspruch 5, wobei der externe Überdruck einen Verband oder eine Bandage um die Gewebeebenen umfasst.

7. Azelluläre Gewebematrix zur Verwendung nach Anspruch 1, weiterhin umfassend ein Vorrichtung zur Therapie mit vermindertem Druck.

8. Azelluläre Gewebematrix zur Verwendung nach Anspruch 7, wobei die Vorrichtung zur Therapie mit vermindertem Druck eine Unterdruckquelle umfasst, die durch einen Fluiddurchlass oder eine Fluidleitung fluidmäßig mit der azellulären Gewebematrix verbunden ist, und wobei die Vorrichtung zur Therapie mit vermindertem Druck einen internen Unterdruck an die azelluläre Gewebematrix abgibt.

9. Azelluläre Gewebematrix zur Verwendung nach Anspruch 8, wobei die azelluläre Gewebematrix an umgebenden Gewebeebenen durch den internen Unterdruck gesichert wird.

10. Azelluläre Gewebematrix zur Verwendung nach Anspruch 8, weiterhin umfassend einen porösen Verteiler, der in oder nahe der implantierten azellulären Gewebematrix positioniert ist, mit der Unterdruckquelle durch den Fluiddurchlass oder die Fluidleitung verbunden ist und Unterdruck innerhalb oder nahe der implantierten azellulären Gewebematrix verteilt.

11. Azelluläre Gewebematrix zur Verwendung nach Anspruch 8, wobei die Unterdruckquelle eine Pumpe ist.

12. Azelluläre Gewebematrix zur Verwendung nach Anspruch 8, weiterhin umfassend ein Abdecktuch, um einen Situs, an dem eine azelluläre Gewebematrix implantiert ist, zu versiegeln.

13. Kit zur Verwendung beim Behandeln eines Patienten, der einer Gewebeannäherung bedarf, um die Bildung eines Seroms oder Hämatoms zu verhindern, umfassend die azelluläre Gewebematrix nach Anspruch 1 und Anweisungen zur Verwendung der azellulären Gewebematrix durch Implantieren der azellulären Gewebematrix zwischen getrennten Gewebeebenen.

14. Kit nach Anspruch 13, weiterhin umfassend eine Vorrichtung zur Therapie mit vermindertem Druck.

## Revendications

1. Matrice tissulaire acellulaire comprenant une matrice extracellulaire d'un tissu décellularisé pour une utilisation dans un procédé pour prévenir la formation d'un sérome ou d'un hématome par rapprochement de tissus séparés par implantation de la matrice tissulaire acellulaire dans un espace entre au moins deux plans tissulaires séparés, la matrice tissulaire acellulaire implantée comprenant une mousse capable de supporter la migration et la prolifération de cellules à partir du tissu environnant après l'implantation.

2. Matrice tissulaire acellulaire pour l'utilisation selon la revendication 1, dans laquelle la matrice tissulaire acellulaire implantée comprend en outre une ou plusieurs cellules viables qui sont histocompatibles avec le patient dans lequel elles sont implantées.

3. Matrice tissulaire acellulaire pour l'utilisation selon la revendication 2, dans laquelle la ou les cellules viables sont des cellules souches.

4. Matrice tissulaire acellulaire pour l'utilisation selon la revendication 1, dans laquelle la matrice tissulaire acellulaire implantée est fixée aux plans tissulaires environnants à l'aide de sutures biodégradables.

5. Matrice tissulaire acellulaire pour l'utilisation selon la revendication 1, dans laquelle la matrice tissulaire acellulaire implantée est fixée aux plans tissulaires environnants par l'application d'une pression externe positive aux plans tissulaires entourant la matrice tissulaire acellulaire implantée.

6. Matrice tissulaire acellulaire pour l'utilisation selon la revendication 5, dans laquelle la pression externe positive comprend un pansement ou une liaison autour des plans tissulaires.

7. Matrice tissulaire acellulaire pour l'utilisation selon la revendication 1, comprenant en outre un dispositif de thérapie sous pression réduite.

8. Matrice tissulaire acellulaire pour l'utilisation selon la revendication 7, dans laquelle le dispositif de thérapie sous pression réduite comprend une source de pression négative qui est reliée fluidiquement par un passage de fluide ou une tubulure à la matrice tissulaire acellulaire et dans laquelle le dispositif de thérapie sous pression réduite délivre une pression interne négative à la matrice tissulaire acellulaire.

9. Matrice tissulaire acellulaire pour l'utilisation selon la revendication 8, dans laquelle la matrice tissulaire acellulaire est fixée aux plans tissulaires environnants par la pression interne négative.

10. Matrice tissulaire acellulaire pour l'utilisation selon la revendication 8, comprenant en outre un distributeur poreux qui est placé dans ou à proximité de la matrice tissulaire acellulaire implantée, est relié à la source de pression négative par le passage de fluide ou la tubulure, et distribue une pression négative dans ou à proximité de la matrice tissulaire acellulaire implantée.

11. Matrice tissulaire acellulaire pour l'utilisation selon la revendication 8, dans laquelle la source de pression négative est une pompe.

12. Matrice tissulaire acellulaire pour l'utilisation selon la revendication 8, comprenant en outre un champ pour sceller de manière étanche un site où une matrice tissulaire acellulaire a été implantée.

13. Kit pour une utilisation dans le traitement d'un patient nécessitant un rapprochement de tissus pour prévenir la formation d'un sérome ou d'un hématome, comprenant la matrice tissulaire acellulaire selon la revendication 1 et des instructions pour utiliser la matrice tissulaire acellulaire par implantation de la matrice tissulaire acellulaire entre des plans tissulaires séparés.

14. Kit selon la revendication 13, comprenant en outre un dispositif de thérapie sous pression réduite.
